# EUROPEAN PATENT APPLICATION

(11) **EP 0 622 463 A2**
(43) Date of publication of application: **02.11.1994**
(21) Application number: 94302946.2
(22) Date of filing: 25.04.1994
(51) Int. Cl.: C12P 7/64, C12N 1/12

(54) **Processes for culturing marine microalgae and producing docosahexaenoic acid using the same**

(30) Priority: 26.04.1993 JP 99656/93
(71) Applicant: KAWASAKI STEEL CORPORATION, Chuo-Ku, Kobe-Shi Hyogo-Ken (JP)
(72) Inventor: Takeuchi, Daizo, c/o Technical Research Division, Chuo-ku, Chiba-shi, Chiba (JP); Uehara, Kenichi, c/o Technical Research Division, Chuo-ku, Chiba-shi, Chiba (JP); Iizuka, Tokio, c/o Technical Research Division, Chuo-ku, Chiba-shi, Chiba (JP)
(74) Representative: Sanderson, Nigel Paul

(57) **Abstract**

This invention provides a process for the stable and low cost production of docosahexaenoic acid having no fishy smells in which docosahexaenoic acid is extracted from cells of a marine microalga produced by a continuous liquid culture process which is effective in producing docosahexaenoic acid in high concentration industrially stably.

Particularly, it provides a process for the culturing of marine microalgae which comprises continuously culturing a marine microalga in a liquid medium under aerated and agitated conditions, thereby producing algal cells of the marine microalga, and a process for the production of docosahexaenoic acid which comprises extracting docosahexaenoic acid from the algal cells produced by the marine microalgae production process.

## Description

This invention relates to a process for the production of a polyunsaturated fatty acid, docosahexaenoic acid (to be referred to as "DHA" hereinafter), which is possessed of noteworthy physiological functions such as a cholesterol reducing effect, an anticoagulant effect and a carcinostatic effect, as well as its brain metabolism-related effects in improving memory and learning capacities, preventing senile dementia and treating Alzheimer disease and its effect as an essential fatty acid for the growth of juvenile fish.
Particularly, it relates to a process for the production of DHA which comprises continuously culturing marine microalgae (marine algae of microscopic forms) efficiently in a large quantity and producing DHA from the thus cultured marine microalgal cells stably with a low cost.

More specifically, this invention relates to a process for the production of marine microalgae which comprises producing algal cells of a marine microalga by continuously culturing said marine microalga under aerated and agitated conditions, and to a process for the production of DHA which comprises continuously culturing a marine microalga under aerated and agitated conditions and extracting DHA from the resulting algal cells.

Though it is known that DHA having various physiologically active functions is contained in fish oil, its practical use has been delayed due to difficulties in separating and extracting it from fish oil. In recent years, studies have been conducted in earnest on the elucidation of the physiologically active functions of DHA and practical application thereof, triggered by the finding of the presence of high concentration DHA in tuna orbit fat and the like among various fish oil sources and by the development of efficient fatty acid purification techniques.

With regard to the purification and use of DHA, a large number of reports have been published, such as on an agent for the prevention and treatment of thrombosis (JP-A-57-183716; the term "JP-A" as used herein means an "unexamined published Japanese patent application), a process for the separation and purification of polyunsaturated fatty acids or esters thereof (JP-A-61-37752), a carcinostatic composition containing a polyunsaturated fatty acid (JP-A-63-258816), a process for the separation and purification of polyunsaturated fatty acid alkyl esters (JP-A-63-8356), a carcinostatic agent containing docosahexaenoyl monoglyceride as an active ingredient (JP-A-1-203322), a brain function enhancing agent containing DHA and its esters as active ingredients (JP-A-1-153629) and a brain function improving composition, a learning capacity increasing agent, a memorial power increasing agent, a dementia preventing agent, a dementia treating agent or a physiologically functional food having brain function improving effects (JP-A-2-49723).

In most cases, DHA or DHA-containing oils are extracted and purified from fish oils such as of tuna, sardine, bonito and the like, but having disadvantages in that such oils cannot be used as stable supply sources due mainly to the migratory behavior of fishes, separation of DHA from other unsaturated fatty acids cannot be made easily by reason of the fish oil composition and the fish oil-specific foul smell cannot be removed easily. As for the production of DHA or DHA-containing oils from other materials than the fish oil, several processes have been proposed such as a process in which a polyunsaturated fatty acid is produced by microbial conversion using a strain belonging to the genus *Mortierella* (JP-A-63-185389), a process in which a polyunsaturated fatty acid enriched oil is produced by a microorganism capable of producing arachidonic acid (JP-A-1-304892), a process in which a polyunsaturated fatty acid-containing lipid is produced from a marine microorganism (JP-A-2-142486), a process in which a polyunsaturated fatty acid is produced by a strain belonging to the genus *Echinosporangium* (JP-A-2-23878) and a process in which a polyunsaturated fatty acid is obtained from culture mixtures of *Echinosporangium transversalie* ATCC 16960 and 18036 (EP-A-355972). There are some other known processes in which microorganisms are used such as a bacterium (Delong, E.F. & Yayanos, A.A.; *Appl. Environ. Microbiol*., vol.51 (4), p. 730, 1986), a fungus *Thraustochytrium* (Haskins, R.H. *et al.; Can.* *J. Microbiol*., vol.10, p.187, 1964), a fungus *Entomophthora* (Tyrrell; *Can*. *J.* *Microbiol*., vol.13, p.755, 1957), a fungus *Japonochytrium* sp. ATCC 28207 (JP-A-1-199588) and algae including Dinoflagellates and Haptophyceae (Joseph, J.D.; *Lipids,* vol.10, p.395, 1975: Nichols, P.D. *et al.*, *Phytochemistry,* vol.23, p.1043, 1984). These processes, however, are based on natural propagation or simple standing culture with no means to enhance propagation of algal cells positively. In addition, being small in DHA productivity, these processes are inferior to the fish oil processes from the viewpoint of industrial applicability.

It is known in general that, among culturing methods of microorganisms, batch culture and fed batch culture are not effective in maintaining a high productivity level due to the accumulation of secondary metabolites and waste materials which inhibit microbial growth, while continuous culture is advantageous from a production efficiency point of view because of its capability to maintain and control propagation activity of microbial phase in a medium at a high level. Such a superiority of continuous culture can be found in a large number of reports on a variety of microbial protein (single cell protein, SCP) production processes and the like. In the case of marine microalgae, however, they are produced by the shear free standing culture in almost all cases, even with no attempts to develop a shaking culture process, because of the sensitivity of algal cells to agitation shear. Because of this, it has been believed that continuous culture could not be applied to marine microalgae.

With regard to the shaking culture of marine microalgae, only a report concerning effects of environmental conditions on the formation of DHA in *Crypthecodinium cohnii* (D.H. Beach *et al.; Biochimica et Biophysica Acta,* vol.316, pp.56 - 65, 1975) and a book (David J. Kyle *et al.; Industrial Application of Single* *Cell* *Oils*, chapter 16, pp.287 - 300, American Oil Chemist's Society, 1992) are known to date, but the DHA productivity by these techniques is too low for its industrial production. Recently, an invention regarding production of DHA by *Crypthecodinium cohnii* MK-8840 (ATCC 40750) and compounds containing the same has been applied (WO 91/11918) disclosing a process for the shake culture of marine microalgae. This invention is a process in which nutrients necessary for the growth of algal cells are limited in order to increase a DHA-containing component in algal cells by sacrificing growth of the cells. However, since this process requires exhaustion of nutrients as an essential factor, it cannot be applied to a continuous culture system for the purpose of further increasing DHA-containing lipid in the algal cells, thus entailing a disadvantage from an industrial production point of view. In consequence, it is necessary to resolve a number of technical problems for the materialization of these prior art processes, and practical industrial production of DHA by these processes, therefore, is considered to be difficult in view of economical profitableness.

In addition, it has been reported that productivity of algal cells can be improved by a prior art continuous culture system, but with decreased content of the product of interest, and that production of secondary metabolites by the continuous culture system is economically disadvantageous in comparison with a batch culture system when microorganisms having small specific growth rates are used (P.F. Stanbury and A. Whitaker, *Principles of Fermentation Technology;* a Japanese version translated by B. Ishizaki and published by Gakkai Shuppan Center, p.238, 1988).

It is known that a marine microalga, *Crypthecodinium cohnii,* propagates itself generally by either asexual or sexual reproduction.

In the case of asexual reproduction, each trophozoite becomes a spherical cell, and its cytoplasm repeats 1 to 3 times of cell division to release 2 to 8 swarmers.
Thereafter, individual numbers of algal cells are multiplied by the cell division of these swarmers.

In the case of sexual reproduction, on the other hand, two swarmers as gametes conjugate each other to form a zygote having 4 flagella. Thereafter, these flagella disappear to form a spherical zygote which undergoes meiosis, and each of the resulting cells becomes a trophozoite to perform its propagation.

Taking note of the asexual life history of this marine microalga *Crypthecodinium cohnii,* the inventors of the present invention have isolated a strain which can perform its propagation stably for a prolonged period of time only by asexual reproduction and found a culture condition under which this strain can propagate stably. The present invention has been accomplished on the basis of these results.

Though DHA has various physiological activities such as carcinostatic effects, its supply and quality are not stable because its material is dependent upon fish oil. In addition, the prior art process requires many purification steps and cost in order to remove the fish oil-specific odor. What is more, the fish oil-specific odor cannot be removed sufficiently even by these purification steps, thus entailing inconvenient applicability of the product. Because of these technical problems remained unsolved, the final product becomes expensive.

In view of the above, it therefore becomes an object of the present invention to provide a process in which a marine microalga which, instead of fish, can be industrially produced and supplied stably with a constant quality is continuously cultured, thereby improving docosahexaenoic acid (DHA) content in algal cells and productivity of the algal cells more sharply than the prior art processes and producing DHA from the algal cells stably with a low cost.

With the aim of overcoming the aforementioned problems involved in the prior art, the inventors of the present invention have conducted intensive studies and succeeded in producing algal cells of a marine microalga in a high density by its continuous culturing instead of the prior art batch culture, and found that the DHA content in the algal cells can be improved sharply by this process. On the basis of these results, the DHA production process of the present invention has been accomplished in which the algal cells produced by the continuous culture system are recovered and DHA is extracted and purified from the cells directly or after drying.

Particularly, in accordance with the present invention, there is provided a process for the culturing of marine microalgae which comprises continuously culturing a marine microalga in a medium under aerated and agitated conditions, thereby producing algal cells of the marine microalga.

Preferably, the marine microalga is *Crypthecodinium cohnii* which, more preferably, is resistant to surface active agents and can withstand its shake culture and continuous culture. Also preferably, the culture medium is supplemented with polyols, pyruvic acid, trioleins and/or amino acids. Also preferably, asexual reproduction stage of *Crypthecodinium cohnii* is used in the continuous culturing.

Another object of the present invention is to provide a process for the production of DHA which comprises extracting DHA from the algal cells produced by the above marine microalgae production process.

Still another object of the present invention is to provide a *Crypthecodinium cohnii* strain which is capable of withstanding its continuous culture under aerated and agitated conditions and of producing DHA.

A further object of the present invention is to provide a *Crypthecodinium cohnii* strain which is resistant to surface active agents and capable of withstanding its shake culture and continuous culture and of producing DHA.

Other objects and advantages of the present invention will be made apparent as the description progresses.

The present invention has been accomplished based on the success in finding and breeding a *Crypthecodinium cohnii* strain which can withstand its continuous culture. This strain was obtained by the repeated single cell isolation of continuous culture-resistant algal strains capable of growing under shake culture and continuous culture conditions from marine microalgae which have been considered to be cultivable only by standing culture, and by the subsequent culture adaptation of these isolates. Effects of the present invention have been attained on the basis of such a unique technical success which is completely different from the prior art techniques that cause reduction of the propagation capacity of algal cells after repetition of their liquid shake culturing.

The microorganisms to be used in the present invention are marine microalgae which can produce DHA, such as *Crypthecodinium cohnii* and the like. These are known algae and can be obtained from stock culture organizations including ATCC (American Type Culture Collection). Illustrative examples of such microalgae include *Crypthecodinium cohnii* ATCC 30021, 30543, 30556, 30571, 30572, 30575, 50051, 50053, 50055, 50056, 50058, 50060 and the like stock culture strains, of which *Crypothecodinium cohnii* ATCC 30021 is particularly preferred.

These algal strains are subjected to repeated single cell isolation and culture adaptation to breed a strain which can withstand continuous culture conditions, and algal cells of the thus bred strain are used in the process of the present invention.

In order to breed such a strain, cultured algal cells are suspended in 1.0 to 3.5% physiological saline to a density of 10² to 10³ cells/ml, spread on an agar medium containing 2 to 4% by weight of glucose and 0.2 to 0.5% by weight of yeast extract and then incubated for 3 to 4 days.

Next, each of the colonies thus formed on the agar medium is transferred into a 300 ml capacity conical flask containing 100 ml of a liquid medium (II) having a composition shown in Examples which has been supplemented with a surface active agent to a final concentration of 0.01 to 1.0 g/liter. The thus inoculated colony is cultured at 24 to 32°C for 2 to 8 days on a shaker at 150 to 250 rpm. The resulting culture mixture is subjected to centrifugation to recover algal cells, and DHA is extracted from the recovered cells with an organic solvent to measure DHA productivity of each algal strain.

Next, the algal cells thus obtained by the shake culture are suspended in 2% physiological saline, and the aforementioned standing culture/shake culture procedure is repeated 10 to 15 times. At each step of the repetition, an algal strain is selected which has a high growth rate, a high surface active agent resistance and a high DHA productivity and is capable of withstanding the shake culture condition. Thereafter, each of the thus selected algal strains is subjected to 5 to 10 repetitions of continuous culture using a small jar fermenter of about 5 liter capacity containing the aforementioned surface active agent-supplemented liquid medium (II), at a temperature of 28 to 32°C, at a medium pH of 7.0, at an agitation speed of 200 to 350 rpm and at an aeration rate of 0.2 to 1.5 vvm. In this way, breeding of an algal strain which has a high growth rate, a high surface active agent resistance and a high DHA productivity and is capable of withstanding both shake culture and continuous culture conditions is achieved.

An agar medium prepared by adding about 2% by weight of agar to 2% physiological saline containing 2 to 4% by weight of glucose and 0.2 to 0.5% by weight of yeast extract is used for the single cell isolation, and a liquid medium for use in the following continuous culture operation is used for the culture adaptation.

In the shake culture and continuous culture for the culture adaptation, a surface active agent or an antifoaming agent is added to the liquid medium to a final concentration of from 0.01 to 1.0 g/liter.

Next, continuous culturing is carried out using the thus obtained continuous culture-resistant algal strain. The following describes conditions for the continuous culturing.

Any medium composition may be used for the culturing of the algal strains of the present invention, provided that they can propagate themselves properly in the medium.

The medium may contain appropriate carbon sources, nitrogen sources, inorganic salts and the like. Illustrative examples of the carbon sources which can be assimilated by the algae of the present invention include organic compounds such as glycerol and the like, carbohydrates such as glucose, fructose, galactose and the like and organic acids such as citric acid, acetic acid and the like.

Further, the addition of polyols, pyruvic acid, triolein and/or amino acids into the liquid medium can increase the amount of the DHA yield. Amino acids available in urea-cycle such as arginine and glutamic acid are considered to be useful to the productivity of the DHA. The preferable amount of the addition of polyols, pyruvic acid, triolein and/or amino acids into the liquid medium is 0.25-10mM.

Concentration of the carbon source in the medium for use in the continuous culturing may be in the range of preferably from 0.1 to 6.0% by weight, more preferably from 0.2 to 3.0% by weight. Such a concentration range is preferable in terms of reducing the amount of residual glucose in the cultured medium to be treated after separation recovery of the algal cells, thus saving the cost of glucose, and of maintaining the DHA yield at a constant level.

Illustrative examples of the nitrogen sources include usually used inorganic nitrogen compounds such as ammonium sulfate, ammonium nitrate and the like and organic nitrogen sources such as peptone, yeast extract, casein hydrolysate, glutamic acid, corn steep liquor and the like. Concentration of the nitrogen source in the medium for use in the continuous culturing may be in the range of preferably from 1/10 to 1/5 of the carbon source contained in the medium.

With regard to the inorganic salts, natural sea water may be the best, but various types of known artificial sea water may also be used, as well as various sodium salts, phosphates, magnesium salts, potassium salts, borates, carbonates and the like.

Also useful are trace amounts of heavy metal salts such as iron salts, manganese salts, cobalt salts, zinc salts, chlorine compounds, bromine compounds and the like. These heavy metal salts are also included in the inorganic salts to be used in the process of the present invention.

As for the culturing method, continuous culture of *Crypthecodinium cohnii* is carried out making use of its propagation by asexual reproduction. In this way, the DHA content in algal cells increases sharply.

The condition of asexual reproduction is to maintain the growth condition not to be reduced nutrition for growth, since sexual reproduction of freshwater Dinoflagellates can be observed in the condition of exhaustion of nutrients[Phycologia, 14 (1) ,1-8(1975)].

Since DHA is accumulated inside the algal cells of the *Crypthecodinium cohnii* strain to be used in the present invention, productivity of DHA can be increased by improving productivity of the algal cells.

In addition, as will be shown later in Examples, the marine microalga, *Crypthecodinium cohnii*, to be used in the present invention does not show decrease in the content of DHA as its metabolite even by the continuous culturing.

A preculture is carried out in order to increase the density of algal cells to a certain level (15 g/liter for instance) necessary for the commencement of the continuous culture. The preculture and continuous culture may be carried out at a temperature of from 15 to 32°C, preferably from 28 to 32°C, and within a neutral range of medium pH. Completion of the culturing may be decided based on the amounts of remaining carbon sources and secreted substances in the medium.

The continuous culture is carried out by controlling medium sugar (glucose) concentration at a certain level which gives proper specific growth rate of the algal strain, preferably at a level of 6.0% by weight or less, more preferably from 0.2 to 3.0% by weight.

Yeast extract is used as a nitrogen source in an amount of from 1/5 to 1/10 of the glucose concentration, and minerals are used according to the composition of medium (I) which will be described later in Examples.

The continuous culture, preferably using a jar fermenter equipped with a sparger and an agitator, may be carried out using a surface active agent or an antifoaming agent selected from fatty acid esters, silicons and the like.

Illustrative examples of the surface active or antifoaming agent include glycerine monofatty acid esters, sorbitan fatty acid esters and silicon base antifoaming agents, such as glycerine monooleate, glycerin monostearate, glycerine monopalmitate, glycerine monolinolate, sorbitan monooleate, Silicon KM-75 and the like.

Preferably, the surface active or antifoaming agent may be used in the medium in an amount of from 0.01 to 1.0 g/liter, in order to prevent foaming without spoiling DHA productivity of the algal cells.

According to the continuous culture of the present invention, a jar fermenter or a large tank is used as a culture vessel, and fresh medium is supplied continuously into the vessel when the algal cell density reached a predetermined level, while simultaneously recovering the cultured medium at a rate equivalent to the medium supply rate. The thus propagated algal cells are collected from the recovered culture medium by centrifugation, filtration or the like means.

With regard to the dilution rate of the supply medium, the continuous culture may be effected at a liquid space velocity of 0.25 hr⁻¹ or less but preferably at 0.1 to 0.2 hr⁻¹ to obtain maximum productivity. The velocity if larger than 0.25 hr⁻¹ would cause washout of the culture medium and spoil stable operation of the continuous culture, because it is higher than the specific growth rate for the asexual reproduction of *Crypthecodinium cohnii.*

Though the dilution rate can be maintained at a constant level during the continuous culture operation, it is preferable to increase the dilution rate gradually, for example, at intervals of 10 hours.

Preferably, the continuous culture may be carried out at a temperature of from 26 to 30°C, at an aeration of from 0.2 to 1.0 vvm and, when agitation impellers are used, at an impeller tip peripheral speed of from 50 to 250 cm/sec.

The continuous culture is effected by feeding a fresh medium and keeping the above culture conditions at constant levels.

As for the medium to be fed continuously, the glucose- and yeast extract-supplemented medium (III) shown in the Examples may be used.

Recovery of the algal cells from the discharged culture medium may be effected by a commonly used centrifugation, filtration or the like means, for example by 10 minutes of centrifugation at 8,000 x g and at 10°C. The thus recovered algal cells are used for the extraction of DHA, as such or after drying them by means of freeze drying or heating and aeration.

Extraction and purification of DHA from the algal cells may be carried out preferably in the presence of a fatty acid oxidation inhibitor such as α-tocopherol or in an atmosphere of an inert gas such as nitrogen. An extraction method shown in the Examples in which an organic solvent system such as methanol/chloroform is used and the purification method of Folch, Lees and Stanley is also useful. The thus partially purified product may be further purified for example by various types of column chromatography or recrystallization.

### EXAMPLES

The following examples are provided to further illustrate the present invention. It is to be understood, however, that the examples are for purpose of illustration only and are not intended as a definition of the limits of the present invention.

Medium compositions and sea water used in the inventive and comparative examples are as follows.

### Medium (I) (Aquamarine, manufactured by Yaesu Yakuhin)

| | | | |
|---|---|---|---|
| MgSO₄·6H₂O | 11.11 g | KBr | 0.10 g |
| CaCl₂·2H₂O | 1.54 g | H₃BO₃ | 0.03 g |
| SrCl₃·6H₂O | 0.04 g | NaF | 0.003 g |
| KCl | 0.69 g | NaCl | 24.53 g |
| NaHCO₃ | 0.20 g | Na₂SO₄ | 4.09 g |

distilled water 1.0 liter
pH 7.0 (adjusted with 1 N HCl)

### Medium (II)

Modified medium (I) supplemented with 2% (v/v) glucose and 0.2% (v/v) yeast extract

### Medium (III)

Modified medium (I) supplemented with 24% (v/v) glucose and 2.4% (v/v) yeast extract

### Medium (IV)

Modified medium (II) supplemented with 0.5 g/l of a surface active agent

### Sea water

Sea water near the shore of Kawasaki-cho, Chuoh-ku, Chiba-shi, Japan

### Inventive Example 1

Cells of *Crypthecodinium cohnii* ATCC 30021 were suspended in 2% physiological saline to a density of 10² to 10³ cells/ml, spread on an agar medium containing 2% by weight of glucose and 0.2% by weight of yeast extract and then incubated for 4 days at 28°C.

Next, each of the colonies thus formed on the agar medium was transferred into a 300 ml capacity conical flask containing 100 ml of medium (IV) which has been prepared by supplementing liquid medium (II) with a surface active agent to a final concentration of 0.5 g/liter. The thus inoculated colony was cultured at 28°C for 5 days on a shaker at 180 rpm. The resulting culture mixture was subjected to centrifugation to recover algal cells, and DHA was extracted from the recovered cells with an organic solvent to measure DHA productivity of each algal strain. In this way, an algal strain having high DHA productivity was selected.

Next, cells of the thus selected algal strain were suspended in 2% physiological saline, and the above standing culture/shake culture procedure was repeated 12 times. At each step of the repetition, an algal strain was selected which showed a high growth rate, a high surface active agent resistance and a high DHA productivity and was capable of withstanding the shake culture condition. Thereafter, each of the thus selected algal strains was subjected to 7 repetitions of continuous culture using a small jar fermenter of 5 liter capacity containing the following liquid medium (IV), at a temperature of 28°C, at a medium pH of 7.0, at an agitation speed of 300 rpm and at an aeration rate of 0.3 vvm. In this way, breeding was effected to obtain an algal strain which has a high growth rate, a high surface active agent resistance and a high DHA productivity and is capable of withstanding both shake culture and continuous culture conditions. The thus obtained continuous culture resistant strain was used in the following culture steps under such conditions that the strain was able to repeat its asexual reproduction.

For use in the following culture experiments, medium (II) was prepared by supplementing the composition of medium (I) with 2% (v/v) of glucose and 0.2% (v/v) of yeast extract and sterilizing the resulting mixture at 120°C for 15 minutes in an autoclave.

A loopful of the cells of *Crypthecodinium cohnii* ATCC 30021 were inoculated into 100 ml of medium (II) having an initial pH of 5.5 to 7.5 to carry out 7 days of standing culture at 28°C. (The thus obtained algal cells are to be referred to as "standing culture algal cells" hereinafter.)

A 10 ml portion of the thus obtained culture medium was inoculated into each of 5 flasks (300 ml capacity) containing 100 ml of medium (II) (pH 6.8) and cultured at 28°C for 5 days on a rotary shaker at 180 rpm. (The thus obtained algal cells are to be referred to as "shake culture algal cells" hereinafter.)

The resulting culture broths in the 5 flasks (500 ml) were combined and inoculated as a seed culture into a 10 liter capacity jar fermenter containing 7 liters of medium (II) (pH 7.0) to carry out jar fermentation at a temperature of 28°C, an agitation speed of 300 rpm and an aeration rate of 0.3 vvm. In this case, 0.75 g of a fatty acid ester base surface active agent, glycerine monooleate, was used as an antifoaming agent.

After commencement of the jar fermentation, samples were collected and dried at 105°C for 5 hours to measure algal cell densities. When the density of algal cells reached 5 g/liter, continuous culture was started by continuously feeding medium (III) at a dilution rate of 0.1 hr⁻¹ under the same culture conditions as the case of the above jar fermentation. The continuous culturing after commencement of the continuous feeding was carried out for 3 days by increasing the dilution rate step by step from the initial rate of 0.1 hr⁻¹ to 0.12, to 0.14 and then to 0.16 hr⁻¹, while adjusting the dilution rate minutely to maintain concentration of the medium glucose within the range of from 0.5 to 3.0% by weight. After commencement of the continuous culturing, algal cells each having a plurality of flagella were not found in the culture medium. (The thus obtained algal cells are to be referred to as "continuous culture algal cells" hereinafter.)

The culture medium discharged from the jar fermenter was subjected to 15 minutes of centrifugation at 8,000 x g and at 10°C to recover algal cells which were subsequently freeze-dried.

A portion of the culture medium was dried at 105°C for 5 hours to find a dry algal cell yield of 15.0 g/liter and a dry algal cell productivity of 1.8 g/hr/liter.

Extraction and purification of DHA from the thus freeze-dried algal cells were carried out in accordance with the commonly used Bligh-Dyer extraction method [*Shin Seikagaku Jikken Koza* (A New Course of Biochemical Experiments), vol.4 Lipid-II, p.9, 1991, Tokyo Kagaku Dojin] in which algal cells are mixed with 5 volumes of methanol/chloroform (1/2) and treated with a blender.

The thus partially purified product was subjected to methylesterification (the just described publication, p.54) in order to determine its quantity by a gas chromatographic analysis using an authentic sample of DHA.

Also, DHA separation was carried out in the usual way by a liquid chromatography (column, ODS; mobile phase, methanol: water = 95:5; flow rate, 1.0 ml/min; detection, RI) to obtain DHA of 99.2% purity and to find its productivity of 0.12 g/l/hr.

Fatty acid composition in the algal cells determined by the gas chromatography is as follows.

| | Algal cells | | |
|---|---|---|---|
| Fatty acid culture | Standing culture | Shake culture | Continuous |
| C 12:0 | 33.4% | 5.7% | 3.0% |
| C 14:0 | 40.2% | 27.5% | 16.2% |
| C 16:0 | 10.9% | 22.7% | 23.7% |
| C 18:0 | 0.9% | 1.6% | 1.8% |
| C 18:1 | 3.3% | 8.0% | 9.1% |
| C 22:6 (DHA) | 11.3% | 34.5% | 45.2% |
| Total | 100.0% | 100.0% | 100.0% |

### Inventive Example 2

Each of the following ATCC strains of a marine microalga, *Crypthecodinium cohnii,* was cultured under the same conditions as described in Inventive Example 1. The results are shown in Table 1 in terms of algal cell yield and DHA yield per hour (ATCC 30021, 3 days of continuous culture; other strains, 1 day of continuous culture).

**Table 1**

| Results of *Crypthecodinium cohnii* continuous culture | | | | | |
|---|---|---|---|---|---|
| *C. cohnii* ATCC No. | Productivities of | | *C. cohnii* ATCC No. | Productivities of | |
| | dry cells (g/hr/l) | DHA (g/hr/l) | | dry cells (g/hr/l) | DHA (g/hr/l) |
| 30021 | 1.80 | 0.12 | 50051 | 0.84 | 0.04 |
| 30543 | 0.84 | 0.05 | 50053 | 0.84 | 0.05 |
| 30556 | 0.78 | 0.04 | 50055 | 0.72 | 0.04 |
| 30571 | 0.84 | 0.05 | 50056 | 0.66 | 0.04 |
| 30572 | 0.72 | 0.04 | 50058 | 0.72 | 0.04 |
| 30775 | 0.66 | 0.04 | 50060 | 0.78 | 0.04 |

### Comparative Example 1 (fed batch culture)

Using *Crypthecodinium cohnii* ATCC 30021 as the test strain, standing culture and shake culture were carried out under the same conditions as described in Inventive Example 1, and 500 ml of the thus obtained culture medium was inoculated as a seed into a 10 liter capacity jar fermenter containing 7 liters of medium (II).

Firstly, batch culture was carried out using the thus inoculated jar fermenter until algal cell density (dry cells) reached 5 g/liter at a temperature of 28°C, a medium pH of 6.8, an agitation speed of 300 rpm and an aeration rate of 0.3 vvm. Thereafter, fed batch culture was carried out by aseptically feeding glucose as the carbon source, yeast extract as the nitrogen source and minerals 60, 80 and 100 hours after the commencement of the batch culture, thereby supplying 10% by weight in total of the carbon source, corresponding amount of the nitrogen source (10% by weight) and respective concentrations of minerals of medium (I). The medium temperature was kept constant at 28°C throughout the fed batch culturing, but the agitation speed was increased stepwise from 300 rpm to 315 rpm 60 hours after the commencement of the batch culture, to 330 rpm after 80 hours and then to 350 rpm after 100 hours. In the same manner, the aeration rate was increased stepwise from 0.3 vvm to 0.35 vvm after 60 hours, to 0.4 vvm after 80 hours and then to 0.5 vvm after 100 hours. As an antifoaming agent, 2.25 g (in total) of sorbitan fatty acid ester was added when required. After 120 hours of the culturing, 26.3 g per liter of dry algal cells were obtained. Algal cells each having a plurality of flagella were found in the finally obtained culture medium.

Extraction and purification of DHA from the resulting dry algal cells were carried out in the same manner as described in Inventive Example 1 to obtain 1.15 g per liter of DHA. Productivities of dry algal cells and DHA were 0.21 g/hr/l and 9.58 mg/hr/l, respectively.

### Comparative Example 2

The algal strain obtained in Comparative Example 1 was batch-cultured in the same manner as described in Inventive Example 1, and continuous medium feeding was started when the algal cell density reached 15 g/l as dry cells at a dilution rate of 0.35 hr⁻¹. However, the continuous culturing with stable cell density lasted for only 12 hours, because the growth rate of algal cells could not match the medium feeding rate.

### Comparative Example 3

The fed batch culture process of Comparative Example 1 was repeated using each of the following ATCC strains of *Crypthecodinium cohnii,* with the results shown in Table 2.

**Table 2**

| Results of *Crypthecodinium cohnii* fed batch culture | | | | | |
|---|---|---|---|---|---|
| *C. cohnii* ATCC No. | Productivities of | | *C. cohnii* ATCC No. | Productivities of | |
| | dry cells (g/hr/l) | DHA (g/hr/l) | | dry cells (g/hr/l) | DHA (g/hr/l) |
| 30021 | 0.21 | 0.01 | 50051 | 0.21 | 0.01 |
| 30543 | 0.19 | 0.009 | 50053 | 0.18 | 0.008 |
| 30556 | 0.16 | 0.008 | 50055 | 0.19 | 0.008 |
| 30571 | 0.18 | 0.009 | 50056 | 0.16 | 0.008 |
| 30572 | 0.19 | 0.01 | 50058 | 0.19 | 0.009 |
| 30775 | 0.19 | 0.009 | 50060 | 0.21 | 0.01 |

### Inventive Example 3

Using *Crypthecodinium cohnii* ATCC 30021, continuous culturing was carried out under the same culture conditions as described in Inventive Example 2, except that the medium was further supplemented with 0.267 mM of arginine. As the result, a DHA productivity of 0.16 g/l was obtained.

### Inventive Example 4

Continuous culturing was carried out under the same culture conditions as described in Inventive Example 3, except that the medium was further supplemented with 4.56 g/l of glycerol. As the result, a DHA productivity of 0.12 g/l was obtained.

### Inventive Example 5

Continuous culturing was carried out under the same culture conditions as described in Inventive Example 3, except that the medium was further supplemented with 5.0 g/l of pyruvic acid. As the result, a DHA productivity of 0.14 g/l was obtained.

Thus, as has been described in the foregoing, according to the process of the present invention, DHA having no fishy smells can be provided stably at a low cost by extracting it from cells of a marine microalga produced by a continuous liquid culture process which is effective in producing high density of the algal cells having high DHA content industrially stably, whereas the prior art DHA production is dependent upon its extraction from fish oils such as of tuna, sardine and the like which, being natural products, are unstable in their productivities and qualities that vary depending on the fishing seasons and areas.

## Claims

1. A process for culturing marine microalgae which comprises continuously culturing a marine microalga in a liquid medium under aerated and agitated conditions, thereby producing algal cells of said marine microalga.

2. The marine microalgae production process according to claim 1 wherein said marine microalga is *Crypthecodinium cohnii.*

3. The marine microalgae production process according to claim 2 wherein said *Crypthecodinium cohnii* is resistant to surface active agents and capable of withstanding shake culture and continuous culture conditions.

4. The marine microalgae production process according to any one of the claims 1 to 3 wherein said liquid medium is supplemented with a polyol, pyruvic acid, a triolein and/or an amino acid.

5. The marine microalgae production process according to any one of the claims 2 to 4 wherein asexual reproduction stage of *Crypthecodinium* *cohnii is used.*

6. A process for producing docosahexaenoic acid which comprises continuously culturing a marine microalga in a liquid medium under aerated and agitated conditions and extracting docosahexaenoic acid from resulting algal cells of said marine microalga.

7. The docosahexanoic acid production process according to claim 6 wherein said marine microalga is *Crypthecodinium cohnii.*

8. The docosahexaenoic acid production process according to claim 7 wherein said *Crypthecodinium cohnii* is resistant to surface active agents and capable of withstanding shake culture and continuous culture conditions.

9. The docosahexaenoic acid production process according to any one of the claims 6 to 8 wherein said liquid medium is supplemented with a polyol, pyruvic acid, a triolein and/or an amino acid.

10. The docosahexaenoic acid production process according to any one of the claims 7 to 9 wherein asexual reproduction stage of *Crypthecodinium cohnii* is used.

11. A *Crypthecodinium cohnii* strain which is capable of withstanding aerated and agitated continuous culture conditions and of producing docosahexaenoic acid.

12. A *Crypthecodinium cohnii* strain which is resistant to surface active agents and capable of withstanding shake culture and continuous culture conditions and of producing docosahexaenoic acid.
